# EUROPEAN PATENT APPLICATION

(11) **EP 2 018 822 A1**
(43) Date of publication of application: **28.01.2009**
(21) Application number: 07742514.8
(22) Date of filing: 26.04.2007
(51) Int. Cl.: A61B 1/00

(54) **NODE RING COUPLER FOR ENDOSCOPE INSERTING PORTION AND METHOD FOR MANUFACTURING THE SAME**

(30) Priority: 17.05.2006 JP 2006138087
(71) Applicant: Olympus Corporation, Tokyo 151-0072 (JP)
(72) Inventor: KITAGAWA, Hideya c/o Intellectual Property Support Department, 2-3 Kuboyama-cho, Hachioji-shi Tokyo 192-8512 (JP); HOSAKA, Kiyokazu c/o Intellectual Property Support Department, 2-3 Kuboyama-cho, Hachioji-shi Tokyo 192-8512 (JP)
(74) Representative: Winter, Brandl, Fürniss, Hübner Röss, Kaiser, Polte Partnerschaft Patent- und Rechtsanwaltskanzlei
(86) International application number: PCT/JP2007/059079
(87) International publication number: WO 2007/132664

(57) **Abstract**

A manufacturing method of a bending part coupling assembly for an endoscope insertion portion having a high manufacturing efficiency is provided.

A manufacturing method of a bending part coupling assembly (22) for an endoscope insertion portion including forming in one plate material (45) having elasticity a bending part coupling assembly preparing portion (66) including a plurality of bending part preparing portions (68) and at least one coupling portion preparing portion (70) by punching in such a manner that the bending part coupling assembly preparing portion (66) is supported by a support portion (81) extending from an edge of the plate material (45), bending at least a portion of the coupling portion preparing portion (70) to cross a plane parallel to the plate material (45), U-bending the bending part coupling assembly preparing portion (66) at least once to form the bending part coupling assembly preparing portion (66) into a cylindrical shape, and O-bending the U-bent bending part coupling assembly preparing portion (66) to form the bending part coupling assembly preparing portion (66) into a cylindrical shape.

## Description

### Technical Field

The present invention relates to a bending part coupling assembly forming a framework structure of an endoscope insertion portion, and a manufacturing method thereof.

### Background Art

A bending portion to be operated to be bent is arranged in an endoscope insertion portion, and this bending portion includes a bending part coupling assembly forming a framework structure thereof. In this bending part coupling assembly, a plurality of cylindrical bending parts are arranged side by side coaxially with respect to each other, and the bending parts adjacent to each other are coupled to swing by a pair of coupling portions provided at positions symmetrical with respect to a central axis. These coupling portions are formed by providing tongue piece portions protruding from both ends of each bending part, superimposing the tongue piece portions of the bending parts adjacent to each other, and riveting them to allow relative rotation thereof.

Further, Jpn. Pat. Appln. KOKAI Publication No. 7-128599 discloses formation of a coupling portion by interposing a steel ball between two superimposed tongue piece portions in place of riveting the tongue piece portions to improve assembling properties of a bending part coupling assembly.

### Disclosure of Invention

However, in a conventional bending part coupling assembly, precise minute components must be very accurately assembled even in the case of such a bending part coupling assembly as disclosed in Jpn. Pat. Appln. KOKAI Publication No. 7-128599. Therefore, manufacture of the bending part coupling assembly is dependent on highly skilled manpower or requires a large-scale high-performance automatic assembling apparatus, and hence improving manufacturing efficiency of the bending part coupling assembly is difficult.

In view of the above-explained problem, it is an object of the present invention to provide a manufacturing method of a bending part coupling assembly for an endoscope insertion portion having a high manufacturing efficiency, and a bending part coupling assembly for an endoscope insertion portion suitable for manufacture based on such a manufacturing method.

In a first aspect of the present invention, a manufacturing method of a bending part coupling assembly for an endoscope insertion portion including a plurality of cylindrical bending parts arranged side by side coaxially with respect to each other and at least one coupling portion coupling the two bending parts adjacent to each other includes: a first step of forming in one plate material having elasticity a bending part coupling assembly preparing portion including a plurality of bending part preparing portions for formation of the plurality of bending parts and at least one coupling portion preparing portion for formation of at least the single coupling portion by punching in such a manner that the bending part coupling assembly preparing portion is supported by a support portion extending from an edge of the plate material; a second step of bending at least a portion of the coupling portion preparing portion to cross a plane parallel to the plate material; a third step of U-bending the bending part coupling assembly preparing portion at least once to form the bending part coupling assembly preparing portion into a cylindrical shape; and a fourth step of O-bending the U-bent bending part coupling assembly preparing portion to form the bending part coupling assembly preparing portion into a cylindrical shape.

In the manufacturing method of the bending part coupling assembly for the endoscope insertion portion, the entire bending part coupling assembly including both ends can be integrally manufactured from one plate material by punching and bending, thereby improving manufacturing efficiency of the bending part coupling assembly.

In a second aspect of the present invention, the entire bending part coupling assembly preparing portion is simultaneously processed in at least one of the first to fourth steps.

In the manufacturing method of the bending part coupling assembly for the endoscope insertion portion, the entire bending part coupling assembly preparing portion is processed at a time and hence a processing time for the bending part coupling assembly can be reduced.

In a third aspect of the present invention, the plate material is fed progressively to processing positions where the respective steps are carried out to be continuously processed in the first to fourth steps.

In the manufacturing method of the bending part coupling assembly for the endoscope insertion portion, the plate material is progressively passed to the processing position where each step is carried out to be continuously processed, thereby improving mass production efficiency of the bending part coupling assembly.

In a fourth aspect of the present invention, the plate material includes a longitudinal axis, and the processing is carried out with a longitudinal direction of the bending part coupling assembly preparing portion crossing a longitudinal direction of the plate material in the first to fourth steps.

In the manufacturing method of the bending part coupling assembly for the endoscope insertion portion, the processing is carried out with the longitudinal direction of the bending part coupling assembly preparing portion crossing the longitudinal direction of the plate material, and an entire length of a manufacturing line can be reduced as compared with an example where the longitudinal direction of the bending part coupling assembly preparing portion is parallel to the longitudinal direction of the plate material.

In a fifth aspect of the present invention, a core material is arranged in the U-bent bending part coupling assembly preparing portion to O-bend the bending part preparing portion along an outer peripheral surface of the core material in the fourth step.

In the manufacturing method of the bending part coupling assembly for the endoscope insertion portion, the core material is used in O-bending, thus improving processing performance of O-bending.

In a sixth aspect of the present invention, the manufacturing method of the bending part coupling assembly for the endoscope insertion portion further includes: a fifth step of joining both abutted side ends of the O-bent bending part preparing portion after the fourth step.

In the manufacturing method of the bending part coupling assembly for the endoscope insertion portion, both side ends of the bending part preparing portion are joined, and the bending part coupling assembly with improved strength can be manufactured as compared with an example where both side ends are not joined.

In a seventh aspect of the present invention, the manufacturing method of the bending part coupling assembly for the endoscope insertion portion further includes: a step of forming a connecting portion preparing portion for formation of a connecting portion connected with a distal end forming portion, a insertion tube portion, or an operating portion of an endoscope in the bending part preparing portion provided in at least one end of the bending part coupling assembly preparing portion before the third step.

In the manufacturing method of the bending part coupling assembly for the endoscope insertion portion, the connecting portion preparing portion is formed in the bending part preparing portion at the end of the bending part coupling assembly preparing portion to form the connecting portion at the end of the bending part coupling assembly. Therefore, additional processing for forming the connecting portion does not have to be performed after U- or O-bending, thereby further improving manufacturing efficiency of the bending part coupling assembly.

In an eighth aspect of the present invention, the manufacturing method of the bending part coupling assembly for the endoscope insertion portion further includes: a step of forming a wire receiver preparing portion for formation of a wire receiver through which a wire is inserted in at least one of the plurality of bending part preparing portions before the third step.

In the manufacturing method of the bending part coupling assembly for the endoscope insertion portion, the wire receiver preparing portion is formed in the at least one of the plurality of bending part preparing portions to form the wiring receiver in the bending part. Therefore, additional processing for forming the wire receiver does not have to be carried out after U- or O-bending, thereby further improving manufacturing efficiency of the bending part coupling assembly.

In a ninth aspect of the present invention, the wire receiver preparing portion is formed in each bending part preparing portion on one end side of the bending part coupling assembly preparing portion and the wire receiver preparing portion is not formed in each remaining bending part preparing portion on the other end side at the step of forming the wire receiver preparing portion.

In the manufacturing method of the bending part coupling assembly for the endoscope insertion portion, the wire receiver preparing portion is formed at each bending part preparing portion on the one end side of the bending part coupling assembly preparing portion and the wire receiver preparing portion is not formed in each remaining bending part preparing portion on the other end side so that the wire receiver is formed in each bending part on the one end side of the bending part coupling assembly and the wire receiver is not formed in each remaining bending part preparing portion on the other end side. Therefore, a bending portion can be formed based on the one end side of the bending part coupling assembly, and an insertion tube portion can be formed based on the other end side. In the manner, the bending part coupling assembly which is entirely integrally manufactured based on punching and bending can be used to form a framework structure of the entire endoscope insertion portion, thereby improving manufacturing efficiency of the endoscope insertion portion.

In a tenth aspect of the present invention, a bending part coupling assembly for an endoscope insertion portion includes: a plurality of cylindrical bending parts arranged side by side coaxially with respect to each other; and at least one coupling portion coupling the two bending parts adjacent to each other, wherein the bending parts and the coupling portion are integrally formed from one plate material having elasticity, the coupling portion includes a hinge portion that is formed in at least a portion of the coupling portion and arranged to cross a cylindrical surface including circumferential surfaces of the two bending parts coupled through the coupling portion to allow relative swing of the two bending parts, the bending part at one end of the bending part coupling assembly includes a distal end side connecting portion connected with a distal end forming portion of an endoscope, and the bending part at the other end of the bending part coupling assembly includes a proximal end side connecting portion connected with a insertion tube portion or an operating portion of the endoscope.

The endoscope bending part coupling assembly is suitable for manufacture based on the manufacturing method of the bending part coupling assembly for the endoscope insertion portion in the first aspect of the present invention.

In an eleventh aspect of the present invention, the bending part includes a discontinuous portion extending in the entire bending part along an axial direction of the bending part.

The endoscope bending part coupling assembly is suitable for manufacture based on the manufacturing method of the bending part coupling assembly for the endoscope insertion portion in the first aspect of the present invention.

In a twelfth aspect of the present invention, the bending part includes a joint portion extending in the bending part along an axial direction of the bending part.

The endoscope bending part coupling assembly is suitable for manufacture based on the manufacturing method of the bending part coupling assembly for the endoscope insertion portion in the sixth aspect of the present invention.

In a thirteenth aspect of the present invention, at least one of the plurality of bending parts includes a wire receiver through which a wire is inserted.

The endoscope bending part coupling assembly is suitable for manufacture based on the manufacturing method of the bending part coupling assembly for the endoscope insertion portion in the eighth aspect of the present invention.

In a fourteenth aspect of the present invention, each bending part on one end side of the bending part coupling assembly includes the wire receiver, and each remaining bending part on the other end side does not include the wire receiver.

The endoscope bending part coupling assembly is suitable for manufacture based on the manufacturing method of the bending part coupling assembly for the endoscope insertion portion in the ninth aspect of the present invention.

In a fifteenth aspect of the present invention, an endoscope includes the above bending part coupling assembly for the endoscope insertion portion.

The endoscope includes the same effect as the bending part coupling assembly for the endoscope insertion portion.

### Brief Description of Drawings

FIG. 1 is a perspective view showing an endoscope according to a first embodiment of the present invention.
FIG. 2 is a perspective view showing a bending part coupling assembly, a distal end forming member, and a connection ring according to the first embodiment of the present invention.
FIG. 3 is a schematic view showing a pressing machine used in the manufacturing method of the bending part coupling assembly according to the first embodiment of the present invention.
FIG. 4A is a top view and a side view for explaining steps 1 to 6 in the manufacturing method of the bending part coupling assembly according to the first embodiment of the present invention.
FIG. 4B is a top view and a side view for explaining steps 7 to 11 in the manufacturing method of the bending part coupling assembly according to the first embodiment of the present invention.
FIG. 4C is a transverse cross-sectional view for explaining O-bending in the manufacturing method of the bending part coupling assembly according to the first embodiment of the present invention.
FIG. 5 is a top view for explaining a manufacturing method of a bending part coupling assembly according to a second embodiment of the present invention.
FIG. 6 is a top view for explaining steps 10 to 16 in a manufacturing method of a bending part coupling assembly according to a third embodiment of the present invention.
FIG. 7A is a transverse cross-sectional view for explaining steps 4, 5, and 9 in the manufacturing method of a bending part coupling assembly according to a modification of the third embodiment of the present invention.
FIG. 7B is a transverse cross-sectional view for explaining O-bending in the manufacturing method of the bending part coupling assembly according to the modification of the third embodiment of the present invention.
FIG. 7C is a transverse cross-sectional view for explaining caulking in the manufacturing method of the bending part coupling assembly according to the modification of the third embodiment of the present invention.

### Best Mode for Carrying Out the Invention

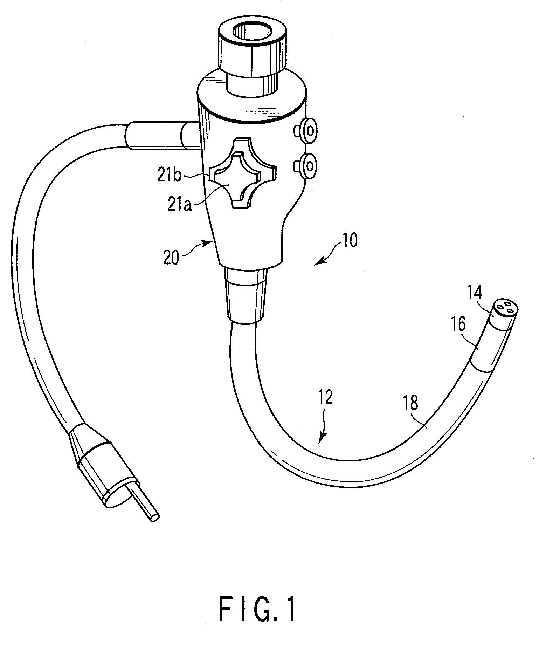

A first embodiment according to the present invention will now be explained with reference to FIGS. 1 to 4C.

As shown in FIG. 1, an endoscope 10 according to the embodiment includes an elongated insertion portion 12 to be inserted into a body cavity. The insertion portion 12 is formed by sequentially coupling a distal end forming portion 14, a bending portion 16 to be operated to be bent, and a long insertion tube portion 18 having flexibility from a distal end side. An operating portion 20 to be held and operated by an operator is coupled with a proximal end of the insertion portion 12, and a vertical bending operation knob 21a and a lateral bending operation knob 21b for bending the bending portion 16 are arranged on the operating portion 20.

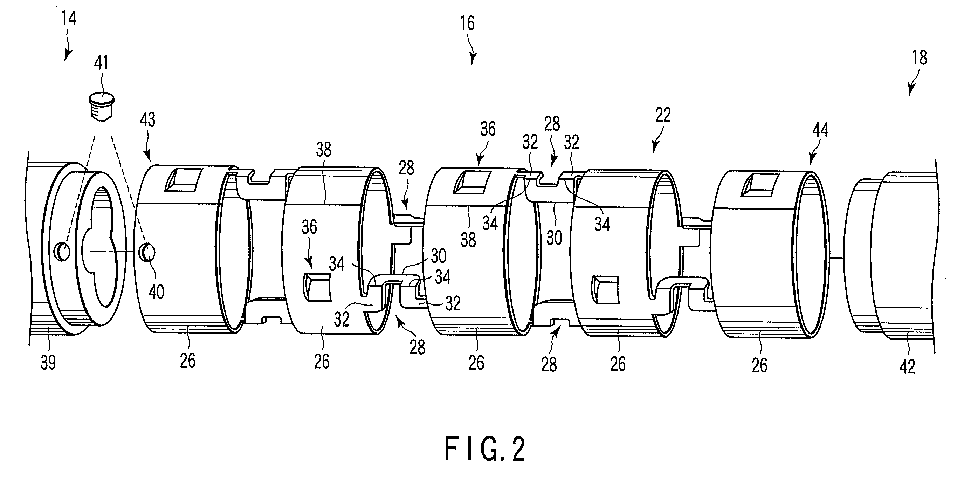

Referring to FIGS. 1 and 2, the bending portion 16 includes bending part coupling assembly 22 forming a framework structure of the bending portion 16. As will be explained later, the entire bending part coupling assembly 22 including both ends respectively connected with a proximal end side of the distal end forming portion 14 and a distal end side of the insertion tube portion 18 is integrally formed from one plate material having flexibility. In this bending part coupling assembly 22, from the distal end forming portion 14 side, first to fifth cylindrical bending parts 26 including a small wall thickness are coaxially arranged side by side, and the bending parts 26 adjacent to each other are coupled with each other by first to fourth pairs of coupling portions 28, each pair of which are placed at symmetrical positions with respect to a central axis of the bending portion 16.

The coupling portion 28 includes a tabular hinge portion 30, and this hinge portion 30 is arranged to be orthogonal to a cylindrical surface including a circumferential surface of each bending part 26 and extends in an axial direction of the bending part coupling assembly 22. Through bending deformation of each hinge portion 30 of each pair of the coupling portions 28 in a substantially central part thereof, the two bending parts 26 coupled with this pair of the coupling portions 28 can swing. The first to fourth pairs of the coupling portions 28 are sequentially shifted and arranged at 90° in the axial direction of the bending part coupling assembly 22 as seen in the central axis direction of the bending part coupling assembly 22. That is, the swing direction of the bending part 26 on the front end side of one bending part 26 is orthogonal to the swing direction of the bending part 26 on the rear end side of the one bending part 26. Combining swings of such bending parts 26 enables the bending part coupling assembly 22 to bend in an arbitrary direction.

Further, the coupling portion 28 that couples the bending parts 26 adjacent to each other includes a pair of holding portions 32 arranged between the hinge portion 30 and the bending part 26. The holding portion 32 is arranged on an end face of the bending part 26 along the above cylindrical surface entirely, extends from the end face of the bending part 26 in the axial direction of the bending part coupling assembly 22, extends in the circumferential direction of the bending part coupling assembly 22, and is coupled with an end of the hinge portion 30 via a bent shape 34. Here, a bend line of the bent shape 34 is parallel to the axial direction of the bending part coupling assembly 22. Therefore, the pair of holding portions 32 are separated from each other, a substantially central part of the hinge portion 30 whose end is coupled through the bent shape 34 thereto is constricted to reduce a width of the tabular shape, and this constricted portion facilitates a bending deformation.

Furthermore, first to fifth pairs of wire receivers 36 through which an operation wire for bending the bending portion 16 is inserted are respectively formed in the first to fifth bending parts 26. These wire receivers 36 have a conformation that each portion between respective slits extending in the circumferential direction and arranged side by side in the bending parts 26 protrudes in a V-like shape toward the inner side in a radial direction. Moreover, the first to fifth pairs of wire receivers 36 are arranged at vertical positions and lateral positions alternately in the axial direction of the bending part coupling assembly 22 with respect to an observation viewing field of the endoscope 10. In the embodiment, the pair of wire receivers 36 formed in each bending part 26 are aligned with formation positions of the coupling portions 28 on the proximal end side of the bending part 26 in the axial direction of the bending part coupling assembly 22. Respective operation wires for vertical and lateral bending operations are inserted through the wire receivers 36 formed at vertical and lateral positions, and the bending portion 16 can be thereby operated to be bent in the vertical and lateral directions.

It is to be noted that, as will be explained later, each bending part 26 is formed by deforming a bending part preparing portion having a long plate shape made of one plate material into a cylindrical shape and abutting both side ends of this portion with each other. Therefore, a discontinuous portion 38 that extends over the entire length of the bending part 26 in the axial direction of the bending part coupling assembly 22 is formed in a cylindrical wall of the bending part 26.

A distal end side of the first bending part 26 at the most distal end is fitted on the proximal end side of a distal end forming member 39 forming the distal end forming portion 14. Additionally, a fixing hole 40 is formed in the cylindrical surface on the distal end side of the first bending part 26, and the distal end side of the first bending part 26 is fixed on the proximal end side of the distal end forming member 39 by a fixing pin 41 disposed in this fixing hole 40. On the other hand, the proximal end side of the fifth bending part 26 at the most proximal end is fitted on the distal end side of a connection ring 42 arranged at the distal end of the insertion tube portion 18. In this manner, in the bending part coupling assembly 22 according to the embodiment, the distal end side of the first bending part 26 at the most distal end forms a distal end side connecting portion 43 connected with the distal end forming portion 14, the proximal end side of the fifth bending part 26 at the most proximal end forms a proximal end side connecting portion 44 connected with the insertion tube portion 18.

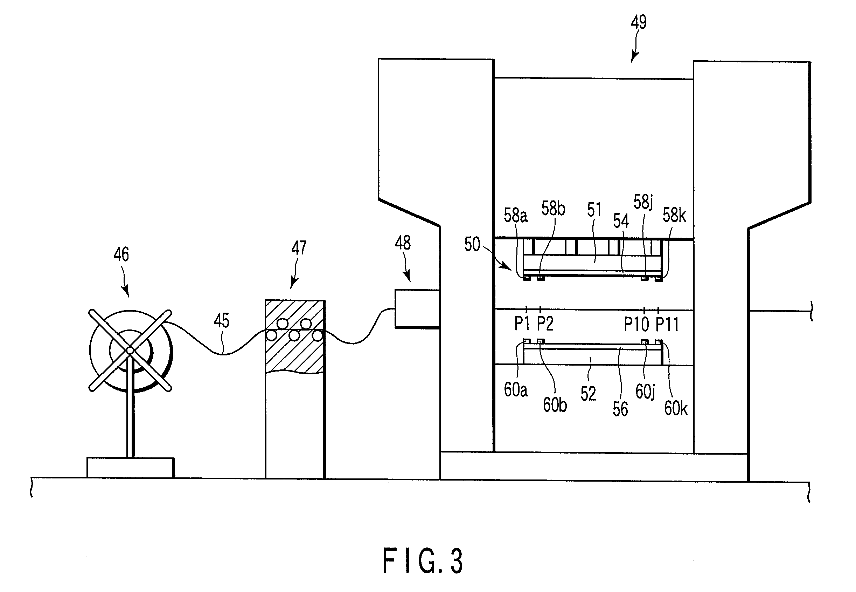

A manufacturing method of the bending part coupling assembly 22 according to the embodiment will now be explained. An outline of the manufacturing method of the bending part coupling assembly 22 will be described with reference to FIG. 3. In manufacturing the bending part coupling assembly 22, a plate material 45 having elasticity is used. As the plate material 45, for example, a metal plate material, e.g., a stainless steel plate material, a spring steel plate material, a non-iron-based spring plate material e.g., phosphorus bronze spring plate material, a resin plate material, or a composite plate material obtained by laminating these plate materials or adding a reinforcing material in these plate materials is used. The plate material 45 is wound around an uncoiler 46. Further, the plate material 45 led out from the uncoiler 46 is straighten into a plane by a lever 47. The plate material 45 led out from the leveler 47 is introduced into a progressive die 50 of a pressing machine 49 by a feeding device 48.

This progressive die 50 is formed of a punch holder 51 as an upper die and a die holder 52 as a lower die. First to eleventh punches 58a, 58b, ..., 58j, 58k are arranged side by side at equal intervals on a punch plate 54 of the punch holder 51, and first to eleventh dies 60a, 60b, ..., 60j, 60k are arranged side by side at equal intervals on a die plate 56 of the die holder 52. The first to eleventh punches 58a, ..., 58k and the first to eleventh dies 60a, ..., 60k respectively face each other to form first to eleventh processing positions P1, P2, ..., P10, P11. The plate material 45 is intermittently fed by a distance corresponding to each gap between the respective processing positions P1, ..., P11 by the feeding device 48, and supplied progressively to the respective processing positions P1, ..., P11 to be continuously processed.

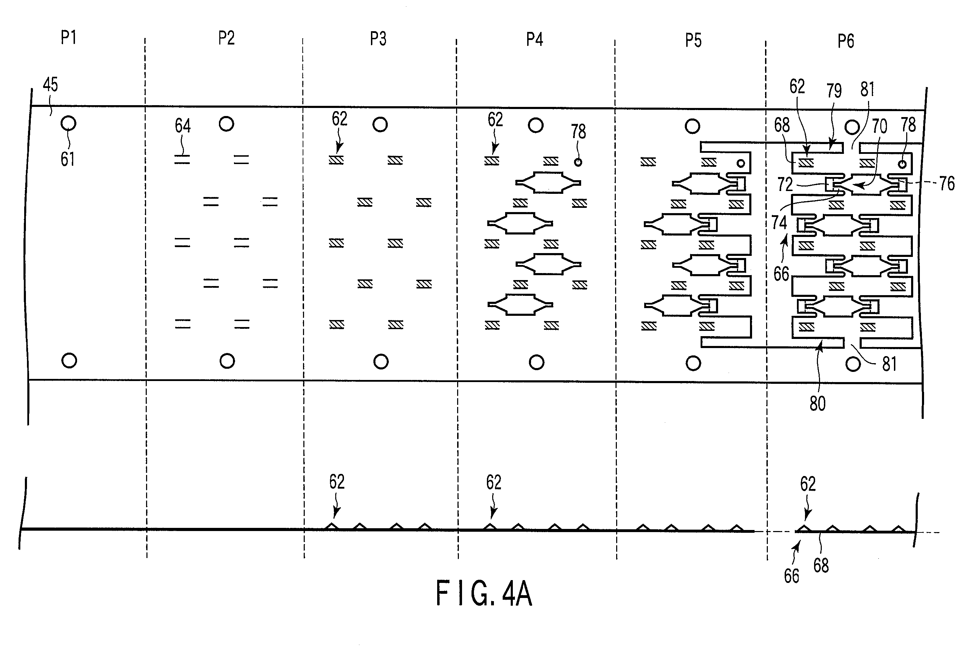

Respective steps in the manufacturing method of the bending part coupling assembly 22 will now be explained in detail with reference to FIGS. 4A to 4C. An entire bending part coupling assembly preparing portion 66 for formation of the bending part coupling assembly 22 is simultaneously processed at the respective processing positions P1, ..., P11. That is, at the respective processing positions P, ..., P11, first to fifth bending part preparing portions 68 for formation of the first to fifth bending parts 26 and first to fourth pairs of coupling portion preparing portions 70 for formation of the first to fourth pairs of coupling portions 28 are simultaneously processed, whilst the plate material 45 is fed progressively. Further, a longitudinal direction of the bending part coupling assembly 22 is orthogonal to a longitudinal direction of the plate material 45, i.e., a feed direction of the plate material 45.

### Step 1 (First Processing position P1)

A pair of pilot holes 61 are formed at both ends with respect to a width direction of the plate material 45. This pair of pilot holes 61 serves for positioning the progressively fed plate material 45 at the respective processing positions P2, ..., P11.

### Step 2 (Second Processing position P2)

In steps 2 and 3, first to fifth pairs of wire receiver preparing portions 62 for formation of the first to fifth pairs of wire receivers 36 are formed.

Here, the two wire receiver preparing portions 62 in one pair of wire receiver preparing portions 62 are arranged side by side to be apart from each other in the longitudinal direction of the plate material 45 by a distance corresponding to a length between the two wire receivers 36 in the circumferential direction of the bending part coupling assembly 22. Furthermore, the first to fifth pairs of wire receiver preparing portions 62 are arranged side by side to be apart from each other in the width direction of the plate material 45 by a length corresponding to a length between pairs of wire receivers 36 adjacent to each other in the axial direction of the bending part coupling assembly 22. Moreover, the second and fourth pairs of wire receiver preparing portions 62 are arranged to be shifted toward the distal end side in the longitudinal direction of the plate material 45 by a length corresponding to 1/2 of the length between the two wire receivers 36 in the circumferential direction of the bending part coupling assembly 22 with respect to the first, third, and fifth pairs of wire receiver preparing portions 62.

In step 2, a pair of slits 64 that respectively define a distal end surface and a proximal end surface of the wire receiver 36 are formed. This pair of slits 64 extend in the longitudinal direction of the plate material 45, and are arranged side by side with respect to each other. Here, a length of the slit 64 in the longitudinal direction of the plate material 45 corresponds to a length of the wire receiver 36 in the circumferential direction of the bending part coupling assembly 22, and a gap between one pair of slits 64 corresponds to a length of the wire receiver 36 in the axial direction of the bending part coupling assembly 22.

### Step 3 (Third Processing position P3)

A portion between one pair of slits 64 is bent into a V-like shape from the side serving as an outer peripheral surface of the bending part 26 toward the side serving as an inner peripheral surface of the same (from a rear side toward a front side of a page space), thereby forming the wire receiver preparing portion 62.

### Steps 4 to 6 (Fourth to Sixth Processing positions P4, P5, and P6)

In the respective steps 4 to 6, in the plate material 45, punching planned portions of the plate material 45 are gradually punched to form the bending part coupling assembly preparing portion 66 for formation of one bending part coupling assembly 22 along the width direction of the plate material 45. That is, in the plate material 45, first to fifth bending part preparing portions 68 for formation of the first to fifth bending parts 26 and first to fourth coupling portion preparing portions 70 for formation of the first to fourth coupling portions 28 are formed along the width direction of the plate material 45.

As seen clearly from a contour outline at the processing position P6, the first to fifth bending part preparing portions 68 respectively have a long plate shape extending in the longitudinal direction of the plate material 45 and are arranged side by side with respect to each other in the width direction of the plate material 45 based on punching in press processing. Moreover, the first to fifth bending part preparing portions 68 respectively includes the first to fifth wire receiving preparing portions 62.

The coupling portion preparing portions 70 are parallel to a plane of the plate material, provided on the plane, and include tabular hinge portion preparing portions 72 extending in the width direction of the plate material 45. Additionally, each coupling portion preparing portion 70 includes a pair of holding portion preparing portions 74 arranged between the bending part preparing portion 68 and the hinge portion preparing portion 72. These holding portion preparing portions 74 are led out in the width direction of the plate material 45 from the bending part preparing portion 68, extend in the longitudinal direction of the plate material 45, and are coupled with the end of the hinge portion preparing portion 72 via a bent shape preparing portion 76. The bent shape preparing portion 76 has a linear shape that is to be bent with respect to the pair of holding portion preparing portions 74 and parallel to the width direction of the plate material 45.

Both coupling portion preparing portions 70 of pair of coupling portion preparing portions 70 are arranged side by side in the longitudinal direction of the plate material 45. Further, each first coupling portion preparing portion 70 between the first and second bending part preparing portions 68 is aligned with each second wire receiver preparing portion 62 of the second bending part preparing portion 68 in the width direction of the plate material 45. Further, each second coupling portion preparing portion 70 between the second and third bending part preparing portions 68 is aligned with each third wire receiver preparing portion 62 of the third bending part preparing portion 68 in the width direction of the plate material 45. Each of the third and fourth coupling portion preparing portions 70 is likewise arranged.

Furthermore, a fixing hole preparing portion 78 for formation of the fixing hole 40 of the first bending part 28 is formed in the first bending part preparing portion 68. Moreover, a distal end side connecting portion preparing portion 79 for formation of the distal end side connecting portion 43 connected with the distal end forming portion 14 is formed on the end side of the first bending part preparing portion 68. Additionally, a proximal end side connecting portion preparing portion 80 for formation of the proximal end side connecting portion 44 connected with the insertion tube portion 18 is formed on the end side of the fifth bending part preparing portion 68.

It is to be noted that, after the sixth step ends, the bending part coupling assembly preparing portion 66 is supported with respect to both ends of the plate material by a pair of support portions 81. The pair of support portions 81 extend inwardly in the axial direction from both ends (both edges) of the plate material 45 in the width direction, and are coupled with central parts of the respective first and fifth bending part preparing portions 68 with respect to the longitudinal direction of the plate material 45 on the end sides of the respective first and fifth bending part preparing portions 68.

### Step 7 (Seventh Processing position P7)

In the bent shape preparing portion 76 of the coupling portion preparing portion 70, the hinge portion preparing portion 72 is bent to be orthogonal to the plane of the plate material 45 from the side serving as the outer peripheral surface of the bending part 26 toward the side serving as the inner peripheral surface thereof (from the rear side toward the front side of a page space). As a result, the hinge portion preparing portion 72 becomes substantially orthogonal to the holding portion preparing portion 74.

### Steps 8 and 9 (Eighth and Ninth Processing positions P8 and P9)

In steps 8 and 9, a line running through the pair of support portions 81 and extending in the width direction of the plate material 45 serve as a bottom line, and the bending part coupling assembly preparing portion 66 is subjected to U-bending from the side serving as the outer peripheral surface of the bending part 26 toward the side serving as the inner peripheral surface thereof (from the rear side toward the front side of a page space) to reach a final curvature from a plane state. It is to be noted that a side end of the planar bending part coupling assembly preparing portion 66 with respect to the longitudinal direction of the plate material 45 is subjected to U-bending in step 8 (first U-bending) and a central portion of the bending part coupling assembly preparing portion 66 with respect to the longitudinal direction of the plate material 45 is subjected to U-bending in step 9 (second U-bending).

### Step 10 (Tenth Processing position P10)

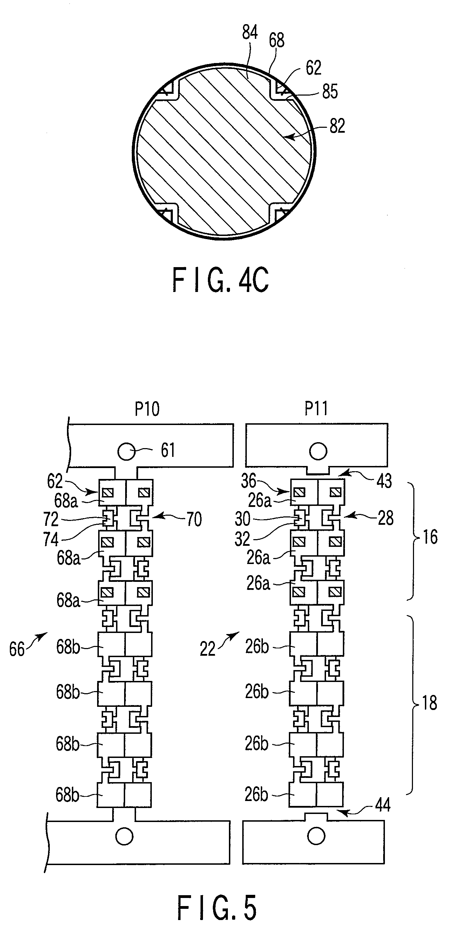

The U-bent bending part coupling assembly preparing portion 66 is further subjected to O-bending to form the cylindrical bending part 26. At this time, each cored bar 82 is respectively protruded inwardly along the width direction from both side ends with respect to the width direction of the plate material 45 so that the cored bar 82 is arranged in the U-bent bending part coupling assembly preparing portion 66, and the bending part coupling assembly preparing portion 66 is subjected to O-bending along an outer peripheral surface of the cored bar 82. As shown in FIG. 4C, the cored bar 82 is formed by extending a groove portion 85 that accommodates the wire receiver preparing portion 62 on an outer peripheral surface portion of a columnar main body portion 84 along a longitudinal direction of the main body portion 84. It is to be noted that both side faces of the bending part preparing portion 68 formed based on O-bending face and about with each other.

### Step 11 (Eleventh Processing position P11)

The pair of support portions 81 formed at both ends with respect to the width direction of the plate material 45 are stricken down to separate the bending part coupling assembly preparing portion 66 from the plate material 45, thereby obtaining the bending part coupling assembly 22.

Therefore, the embodiment includes the following effect.

In the manufacturing method of the bending part coupling assembly 22 according to the embodiment, the bending part coupling assembly preparing portion 66 is formed in one plate material 45 having elasticity based on punching in such a manner that it is supported by the pair of support portions 81 extending from both ends of the plate material 45 with respect to the width direction. Here, the bending part coupling assembly preparing portion 66 includes the first to fifth bending part preparing portions 68 for formation of the first to fifth bending parts 26 and the first to fourth pairs of coupling portion preparing portions 70 for formation of the first to fourth pairs of coupling portions 28. Further, the hinge portion preparing portion 72 of the coupling portion preparing portion 70 is bent to be orthogonal to the plane parallel to the plate material 45. Furthermore, to form the bending part coupling assembly preparing portion 66 into a cylindrical shape, the bending part coupling assembly preparing portion 66 is subjected to U-bending twice and further subjected to O-bending. As explained above, in the manufacturing method of the bending part coupling assembly 22 according to the embodiment, the entire bending part coupling assembly 22 including both ends respectively connected with the proximal end side of the distal end forming portion 14 and the distal end side of the insertion tube portion 18 can be integrally manufactured from the single plate material 45 by press processing alone, thereby improving manufacturing efficiency of the bending part coupling assembly 22.

Here, the bending part coupling assembly 22 according to the embodiment includes the first to fifth cylindrical bending parts 26 that are coaxially arranged side by side with respect to each other and the first to fourth coupling portions 28 that couple the bending parts 26 adjacent to each other, and these bending parts 26 and coupling portions 28 are integrally formed from the single plate material 45 having elasticity. Moreover, each bending part 26 includes the discontinuous portion 38 extending in each entire bending part 26 along the axial direction of the bending part 26. Additionally, each coupling portion 28 includes the hinge portion 30, and this hinge portion 30 is formed on each of both end sides of the bending parts 26 adjacent to each other and arranged to cross a cylindrical surface having circumferential surfaces of the bending parts 26 coupled with each other through the coupling portion 28, thereby allowing relative swing of the bending parts 26. Further, the bending part 26 provided at the most distal end of the bending part coupling assembly 22 includes the distal end side connecting portion 43 connected with the distal end forming portion 14, and the bending part 26 provided at the most proximal end of the bending part coupling assembly 22 includes the proximal end side connecting portion 44 connected with the insertion tube portion 18. Such a bending part coupling assembly 22 is suitable for manufacture based on the manufacturing method of the bending part coupling assembly 22 according to the embodiment.

Further, at the processing positions P1, ..., P11, the entire bending part coupling assembly preparing portion 66 is simultaneously processed, thereby reducing a processing time for the bending part coupling assembly 22.

Furthermore, the plate material 45 is fed progressively to the processing positions P1, ..., P11 where the respective steps are carried out to be continuously processed, thus improving mass production efficiency of the bending part coupling assembly 22.

Moreover, the longitudinal direction of the bending part coupling assembly preparing portion 66 is orthogonal to the longitudinal direction of the plate material 45, and an entire length of a manufacturing line can be shortened as compared with an example where the longitudinal direction of the bending part coupling assembly preparing portion 66 is parallel to the longitudinal direction of the plate material 45.

Additionally, the cored bar 82 is arranged in the U-bent bending part coupling assembly preparing portion 66, the bending part preparing portion 68 is subjected to O-bending along the outer peripheral surface of the cored bar 82, and hence the processing performance of O-bending is improved.

Further, at the time of striking down the pair of support portions 81, the distal end side connecting portion 43 connected with the distal end forming portion 14 has been formed at one end of the bending part coupling assembly 22 and the proximal end connecting portion 44 connected with the insertion tube portion 18 has been formed at the other end of the bending part coupling assembly 22 by forming the distal end side connecting portion preparing portion 79 in the bending part 26 at one end of the bending part coupling assembly preparing portion 66 and the proximal end side connecting portion preparing portion 80 in the bending part 26 at the other end of the bending part coupling assembly preparing portion 66. As explained above, additional processing for formation of the distal end connecting portion 43 and the proximal end connecting portion 44 does not have to be carried out after press working, thus further improving manufacturing efficiency of the bending part coupling assembly 22.

Furthermore, each wire receiver 36 is formed in the bending part 26 by forming each wire receiver preparing portion 62 in the bending part preparing portion 68 based on slit processing and bending. In this manner, additional processing for formation of the wire receiver 36 does not have to be performed after press processing, thereby further improving manufacturing efficiency of the bending part coupling assembly 22.

Here, the bending part 26 of the bending part coupling assembly 22 according to the embodiment includes the wire receiver 36, and such a bending part coupling assembly 22 is suitable for manufacture based on the manufacturing method of the bending part coupling assembly 22 according to the embodiment.

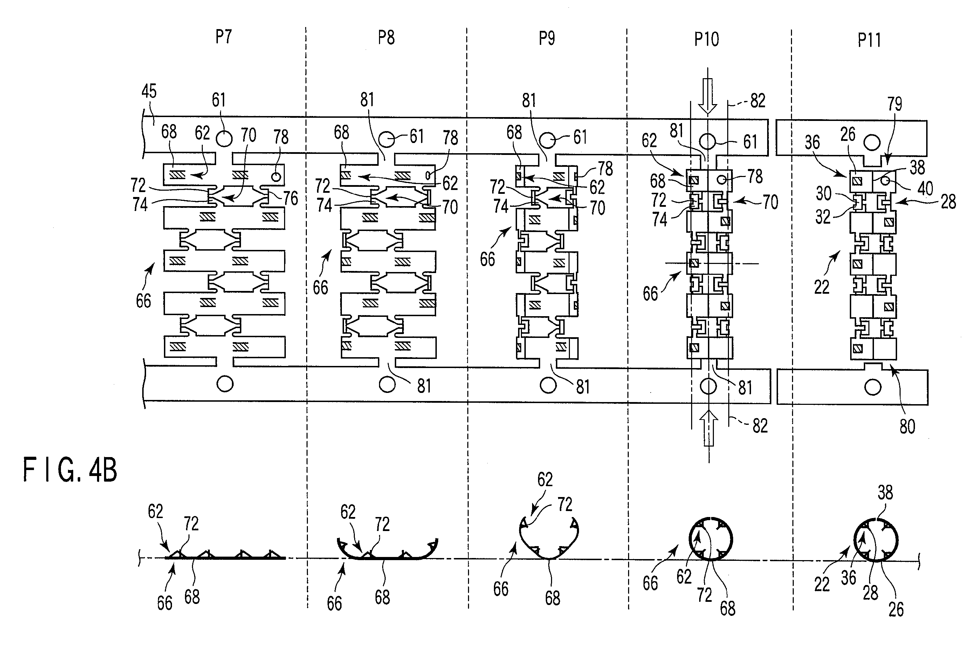

FIG. 5 shows a second embodiment according to the present invention. Like reference numerals denote structures having the same functions as those in the first embodiment, thereby omitting an explanation. FIG. 5 shows steps corresponding to steps carried out at the processing positions P10 and P11 depicted in FIG. 4B.

The bending part coupling assembly 22 according to the embodiment is formed of seven bending parts 26a and 26b and six pairs of coupling portions 28 at the processing position P11. Moreover, wire receivers 36 are formed in the three bending parts 26a on the distal end side, and these three bending parts 26a form the framework structure of the bending portion 16. The distal end side of the bending part 26a provided at the most distal end is fitted on and fixed to the proximal end side of the distal end forming member 39, thereby forming the distal end side connecting portion 43. It is to be noted that the fixing hole 40 is not formed in the distal end side connecting portion 43 according to the embodiment. On the other hand, the wire receivers 36 are not formed in the four bending parts 26b on the proximal end side, and these four bending parts 26b form the framework structure of the insertion tube portion 18. The proximal end side of the bending part 26b provided at the most proximal end is connected with the distal end of the operating portion 20 of the endoscope 10, thereby forming the proximal end side connecting portion 44.

In the manufacturing method of the bending part coupling assembly 22 according to the embodiment, the entire bending part coupling assembly preparing portion 66 including first to seventh bending part preparing portions 68a and 68b and first to sixth pairs of coupling portion preparing portions 70 is simultaneously processed. The manufacturing method of the bending part coupling assembly 22 according to the embodiment is substantially the same as the manufacturing method of the bending part coupling assembly according to the first embodiment. That is, after forming the pilot hole 61 in the first step, wire receiver preparing portions 62 are formed at positions corresponding to the first to third bending part preparing portions 68a alone and the wire receiver preparing portions 62 are not formed at positions corresponding to the fourth to seventh bending part preparing portions 68b, in the second and third steps. Subsequent steps are the same as those in the first embodiment.

Therefore, the embodiment includes the following effect.

In the manufacturing method of the bending part coupling assembly 22 according to the embodiment, the wire receiver preparing portion 62 is formed in each bending part preparing portion 68a on one end side of the bending part coupling assembly preparing portion 66 and the wire receiver preparing portion 62 is not formed in the bending part preparation portions 68b on the other end side so that the wire receiver 36 is formed in each bending parts 26a on one end side of the bending part coupling assembly 22 and the wire receiver 36 is not formed in the remaining bending parts 26b on the other end side. As the result, one end side of the bending part coupling assembly 22 enables forming the bending portion 16, and the other end side of the same enables forming the insertion tube portion 18. In this manner, using the bending part coupling assembly 22 that is entirely integrally manufactured from one plate material by press processing alone enables forming the entire framework structure of the endoscope insertion portion 12, thereby improving manufacturing efficiency of the endoscope insertion portion 12.

Here, bending parts 26 on the distal end side of the bending part coupling assembly 22 according to the embodiment includes the wire receiver 36, and remaining bending parts 26 on the proximal end side does not include the wire receiver 36. Therefore, the bending part coupling assembly 22 according to the embodiment is suitable for manufacture based on the manufacturing method of the bending part coupling assembly 22 according to the embodiment.

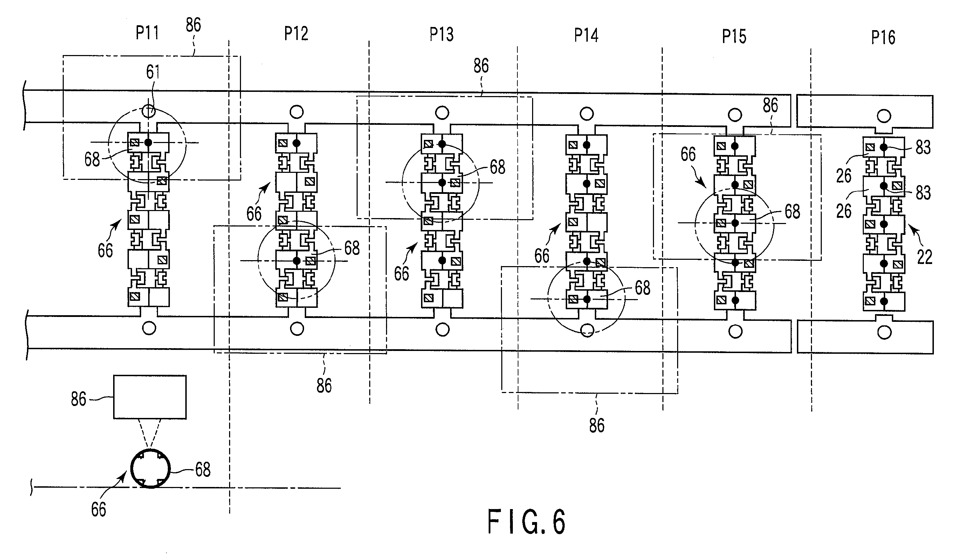

FIG. 6 shows the third embodiment according to the present invention. Like reference numerals denote structures having the same functions as those in the first embodiment, thereby omitting an explanation. FIG. 6 is the explanatory drawing of steps after the step carried out at the processing position P10 depicted in FIG. 4B.

The bending part 26 of the bending part coupling assembly 22 according to the embodiment is formed by deforming the bending part preparing portion 68 having the long plate shape into the cylindrical shape, and abutting and joining both side ends thereof with each other as will be explained later in detail. Therefore, the joint portion 83 extending in the axial direction of the bending part 26 is formed in each bending part 26.

Respective steps in the manufacturing method of the bending part coupling assembly 22 according to the embodiment will now be explained in detail with reference to FIG. 6.

### Steps 1 to 10

Based on the same procedure as in steps 1 to 10 in the first embodiment, the O-bent bending part coupling assembly preparing portion 66 is formed wherein both side ends of the bending part preparing portion 68 are abutted with each other.

### Steps 11 to 15 (Eleventh to Fifteenth Processing positions P11, ..., P15)

In steps 11 to 15, abutted portion formed of both side ends of each of the first to fifth bending part preparing portions 68 in the first to fifth bending part preparing portions 68 are sequentially jointed.

Specifically, the laser emission unit 86 is arranged at each of the eleventh to fifteenth processing positions P11, ..., P15, and the abutted portions are subjected to laser welding in the order of the first, fourth, second, fifth, and third bending part preparing portions 68 in steps 11 to 15.

### Step 16 (Sixteenth Processing position)

As in step 11 in the first embodiment, the pair of support portions 81 formed at both ends with respect to the width direction of the plate material 45 is stricken down to separate the bending part coupling assembly preparing portion 66 from the plate material 45, thereby obtaining the bending part coupling assembly 22.

Therefore, the embodiment includes the following effect.

In the manufacturing method of the bending part coupling assembly 22 according to the embodiment, both side ends of the bending part preparing portion 68 subjected to O-bending to be abutted with each other are joined. Therefore, as compared with the example where the abutted portions in the bending part preparing portion 68 are not joined, the bending part coupling assembly 22 having improved strength can be manufactured.

Here, each bending part 26 in the bending part coupling assembly 22 according to the embodiment includes the joint portion 83 extending in each bending part 26 in the axial direction of the bending part 26, and it is suitable for manufacture based on the manufacturing method of the bending part coupling assembly 22 according to the embodiment.

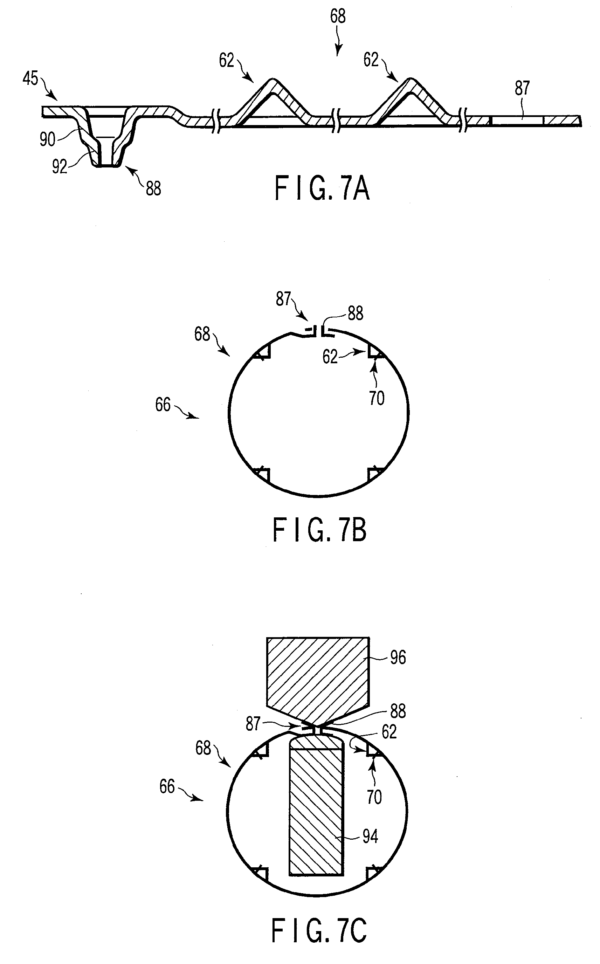

FIGS. 7A to 7C show the modification of the third embodiment according to the present invention. Like reference numerals denote structures having the same functions as those in the first embodiment, thereby omitting the explanation.

Both side ends of the bending part 26 in the bending part coupling assembly 22 according to the embodiment are joined by caulking.

Respective steps in the manufacturing method of the bending part coupling assembly 22 according to the embodiment will now be explained in detail with reference to FIGS. 7A to 7C.

### Steps 1 to 3

As in steps 1 to 3 in the first embodiment, the pair of pilot holes 61 and the pair of wire receiver preparing portions 62 are formed in the plate material 45.

### Step 4 (see FIG. 7A)

A joint hole preparing portion 87 is formed by punching on the distal end side of the pair of wire receiver preparing portions 62 in the longitudinal direction of the plate material 45. This joint hole preparing portion 87 will be arranged at one side end of the bending part preparing portion 68 having the long plate shape.

### Step 5 (see FIG. 7A)

A joint protrusion preparing portion 88 formed of a large-diameter portion 90 on the proximal end side and a small-diameter portion 92 on the terminal end side is formed by burring on the rear end side of the pair of wire receiver preparing portions 62 with respect to the longitudinal direction of the plate material 45. This joint protrusion preparing portion 88 will be arranged at the position of the other side end of the bending part preparing portion 68 having the long plate shape corresponding to the joint hole preparing portion 87.

### Steps 6 to 8

As in steps 4 to 6 in the first embodiment, the bending part preparing portion 68 and the coupling portion preparing portion 70 are formed by punching.

### Step 9 (see FIG. 7A)

The other side end of the bending part preparing portion 68 where the joint protrusion preparing portion 88 is arranged is displaced by the amount corresponding to the thickness of the plate material 45 from the side serving as the outer peripheral surface of the bending part 26 toward the side serving as the inner peripheral surface of the same (from the rear side toward the front side of the page space) by step-bending exposing to the bending part preparing portion 68.

### Steps 10 to 12

As in steps 7 to 9 in the first embodiment, bending of the hinge portion preparing portion 72 in the coupling portion preparing portion 70 and U-bending of the bending part coupling assembly preparing portion 66 are carried out.

### Step 13 (see FIG. 7B)

As in step 10 in the first embodiment, O-bending is performed. At this time, the other side end of the bending part preparing portion 68 displaced by step-bending is superimposed on the inner side of the one side end of the bending part preparing portion 68, and the joint protrusion preparing portion 88 on the other side end is fitted in the joint hole preparing portion 87 on one side end.

### Steps 14 to 18 (see FIG. 7C)

In steps 14 to 18, both side ends of the bending part preparing portion 68 are joined by caulking at respective processing positions in the order of the first, fourth, second, fifth, and third bending part preparing portions 68. That is, each cored bar die 94 is protruded inwardly in the width direction from both side ends with respect to the width direction of the plate material 45 so that the cored bar die 94 is arranged in the O-bent bending part coupling assembly preparing portion 66. The cored bar die 94 is used to support the proximal end side of the joint protrusion preparing portion 88 from the inner side of the bending part preparing portion 68, and the conical punch 96 is pushed into the small-diameter portion 92 at the terminal end of the joint protrusion preparing portion 88 from the outer side of the bending part preparing portion 68 to open the small-diameter portion 92. As the result, the joint protrusion preparing portion 88 at the other side end of the bending part preparing portion 68 is engaged with one side end of the bending part preparing portion 68. In this manner, both side ends of the bending part preparing portion 68 are joined.

### Step 19

As in step 11 in the first embodiment, the pair of support portions 81 formed at both ends with respect to the width direction of the plate material 45 are stricken down to separate the bending part coupling assembly preparing portion 66 from the plate material 45, thereby obtaining the bending part coupling assembly 22.

Besides, resistance welding, the adhesive, and others may be used to join both side ends of the bending part coupling assembly preparing portion 66.

Although the bending part coupling assembly 22 including a number of the bending parts 26 and the pair of coupling portions 28 has been explained in the foregoing embodiment, the numbers of the bending parts 26 and the pair of coupling portions 28 are appropriately selected in accordance with the desired length of the bending portion 16 or the insertion tube portion 18.

Further, the connecting portion formed at the end of the bending part coupling assembly 22 has the various kinds of conformations, e.g., the soldering hole, the locking hole, the groove, the protrusion, and others in accordance with conformations of the connection member of the distal end forming portion 14, the insertion tube portion 18 or the operating portion 20.

Furthermore, the number of times of U- or O-bending may be increased or decreased in accordance with the bending amount.

Moreover, the number of the wire receivers formed in each bending part in the bending portion is not restricted to two, and the four wire receivers may be formed at equal intervals in the circumferential direction of the bending part. Although the wire receiver preparing portion 62 for formation of the wire receiver is formed by bending the portion between the slits 64 into the V-like shape in each embodiment, the present invention is not restricted thereto, and this portion may be bend into the arc shape.

Additionally, the U-like shape described in U-bending in each of the embodiments according to the present invention is not restricted to the shape having both sides parallel to each other and the bottom having the semi-arc shape, and any shape is adopted as long as it has both side surfaces and the bottom surface and the opened upper portion.

Further, the O-like shape described in O-bending in each of the embodiments according to the present invention is not restricted to the shape having both side faces of the bending part preparing portion abutted to each other, and may be the substantially cylindrical shape where both side faces are slightly apart from each other due to elasticity of the plate material forming the bending part preparing portion.

Furthermore, punching of the plate material is not restricted to punching based on press processing, and punching may be carried out by irradiating the energy line of, e.g., the laser processing device whose movement with respect to the plate material is NC-controlled. It is to be noted that manufacturing the bending part coupling assembly by pressing processing alone is preferable in terms of cost.

## Claims

1. A manufacturing method of a bending part coupling assembly for an endoscope insertion portion including a plurality of cylindrical bending parts arranged side by side coaxially with respect to each other and at least one coupling portion coupling the two bending parts adjacent to each other, the method comprising:
a first step of forming in one plate material having elasticity a bending part coupling assembly preparing portion including a plurality of bending part preparing portions for formation of the plurality of bending parts and at least one coupling portion preparing portion for formation of at least the single coupling portion by punching in such a manner that the bending part coupling assembly preparing portion is supported by a support portion extending from an edge of the plate material;
a second step of bending at least a portion of the coupling portion preparing portion to cross a plane parallel to the plate material;
a third step of U-bending the bending part coupling assembly preparing portion at least once to form the bending part coupling assembly preparing portion into a cylindrical shape; and
a fourth step of O-bending the U-bent bending part coupling assembly preparing portion to form the bending part coupling assembly preparing portion into a cylindrical shape.

2. The manufacturing method of the bending part coupling assembly for the endoscope insertion portion according to claim 1,
wherein the entire bending part coupling assembly preparing portion is simultaneously processed in at least one of the first to fourth steps.

3. The manufacturing method of the bending part coupling assembly for the endoscope insertion portion according to claim 2,
wherein the plate material is fed progressively forwards to processing positions where the respective steps are carried out to be continuously processed in the first to fourth steps.

4. The manufacturing method of the bending part coupling assembly for the endoscope insertion portion according to claim 3,
wherein the plate material includes a longitudinal axis, and
the processing is carried out with a longitudinal direction of the bending part coupling assembly preparing portion crossing a longitudinal direction of the plate material in the first to fourth steps.

5. The manufacturing method of the bending part coupling assembly for the endoscope insertion portion according to claim 1,
wherein a core material is arranged in the U-bent bending part coupling assembly preparing portion to O-bend the bending part preparing portion along an outer peripheral surface of the core material in the fourth step.

6. The manufacturing method of the bending part coupling assembly for the endoscope insertion portion according to claim 1, further comprising:
a fifth step of joining both abutted side ends of the O-bent bending part preparing portion after the fourth step.

7. The manufacturing method of the bending part coupling assembly for the endoscope insertion portion according to claim 1, further comprising:
a step of forming a connecting portion preparing portion for formation of a connecting portion connected with a distal end forming portion, a insertion tube portion, or an operating portion of an endoscope in the bending part preparing portion provided in at least one end of the bending part coupling assembly preparing portion before the third step.

8. The manufacturing method of the bending part coupling assembly for the endoscope insertion portion according to claim 1, further comprising:
a step of forming a wire receiver preparing portion for formation of a wire receiver through which a wire is inserted in at least one of the plurality of bending part preparing portions before the third step.

9. The manufacturing method of the bending part coupling assembly for the endoscope insertion portion according to claim 8,
**characterized in that** the wire receiver preparing portion is formed in each bending part preparing portion on one end side of the bending part coupling assembly preparing portion and the wire receiver preparing portion is not formed in each remaining bending part preparing portion on the other end side thereof at the step of forming the wire receiver preparing portion.

10. A bending part coupling assembly for an endoscope insertion portion comprising:
a plurality of cylindrical bending parts arranged side by side coaxially with respect to each other; and
at least one coupling portion coupling the two bending parts adjacent to each other,
wherein the bending parts and the coupling portion are integrally formed from one plate material having elasticity,
the coupling portion includes a hinge portion that is formed in at least a portion of the coupling portion and arranged to cross a cylindrical surface including circumferential surfaces of the two bending parts coupled through the coupling portion to allow relative swing of the two bending parts,
the bending part at one end of the bending part coupling assembly includes a distal end side connecting portion connected with a distal end forming portion of an endoscope, and
the bending part at the other end of the bending part coupling assembly includes a proximal end side connecting portion connected with a insertion tube portion or an operating portion of the endoscope.

11. The bending part coupling assembly for the endoscope insertion portion according to claim 10,
wherein the bending part includes a discontinuous portion extending in the entire bending part along an axial direction of the bending part.

12. The bending part coupling assembly for the endoscope insertion portion according to claim 10,
wherein the bending part includes a joint portion extending in the bending part along an axial direction of the bending part.

13. The bending part coupling assembly for the endoscope insertion portion according to claim 10,
wherein at least one of the plurality of bending parts includes a wire receiver through which a wire is inserted.

14. The bending part coupling assembly for the endoscope insertion portion according to claim 13,
wherein each bending part on one end side of the bending part coupling assembly includes the wire receiver, and each remaining bending part on the other end side does not include the wire receiver.

15. An endoscope comprising a bending part coupling assembly for the endoscope insertion portion including:
a plurality of cylindrical bending parts arranged side by side coaxially with respect to each other; and
at least one coupling portion coupling the two bending parts adjacent to each other,
wherein the bending parts and the coupling portion are integrally formed from one plate material having elasticity,
the coupling portion includes a hinge portion that is formed in at least a portion of the coupling portion and arranged to cross a cylindrical surface including circumferential surfaces of the two bending parts coupled through the coupling portion to allow relative swing of the two bending parts,
the bending part at one end of the bending part coupling assembly includes a distal end side connecting portion connected with a distal end forming portion of an endoscope, and
the bending part at the other end of the bending part coupling assembly includes a proximal end side connecting portion connected with a insertion tube portion or an operating portion of the endoscope.

16. The endoscope according to claim 15,
wherein the bending part includes a discontinuous portion extending in the entire bending part along an axial direction of the bending part.

17. The endoscope according to claim 15,
wherein the bending part includes a joint portion extending in the bending part along an axial direction of the bending part.

18. The endoscope according to claim 15,
wherein at least one of the plurality of bending parts includes a wire receiver through which a wire is inserted.

19. The endoscope according to claim 18,
wherein each bending part on one end side of the bending part coupling assembly includes the wire receiver, and each remaining bending part on the other end side does not include the wire receiver.
